# EUROPEAN PATENT APPLICATION

(11) **EP 1 239 285 A1**
(43) Date of publication of application: **11.09.2002**
(21) Application number: 02004921.9
(22) Date of filing: 05.03.2002
(51) Int. Cl.: G01N 33/536, G01N 33/543, G01N 33/72

(54) **Improved homogeneous immunoassay method**

(30) Priority: 07.03.2001 EP 01105307
(71) Applicant: Roche Diagnostics GmbH, 68305 Mannheim (DE); F. HOFFMANN-LA ROCHE AG, 4070 Basel (CH)
(72) Inventor: Vogt, Bernd, Dr., 82327 Tutzing (DE); Karl, Johann, Dr., 82380 Peissenberg (DE); Mayr, Anita, 82327 Traubing (DE)

(57) **Abstract**

The present invention relates to an improved, homogeneous assay method for detection of an analyte in a sample based on the analyte-dependent aggregation or agglutination of a multivalent carrier reagent, an analyte-derivative bound or capable of binding to said carrier and an analyte-specific binding partner (R1), characterized in that said binding partner (R1) is oligomeric or is oligomerized by a reagent (R2) having at least two binding sides for (R1).

## Description

The present invention relates to an improved, homogeneous assay method for detection of an analyte in a sample based on the analyte-dependent aggregation or agglutination of a multivalent carrier reagent, an analyte-derivative bound or capable of binding to said carrier and an analyte-specific binding partner (R1). It especially relates to assays wherein the binding partner (R1) is oligomeric or is oligomerized by a reagent (R2) having at least two binding sides for (R1).

Binding assays, especially immunological binding assays, nowadays are widely used in research settings as well as in clinical and/or diagnostic routine laboratories.

A large variety of analytes present in quite different ranges of concentrations have to be reliably measured by these procedures.

The different immunological methods of determination can be divided into homogeneous and heterogeneous procedures. A solid phase reaction is always part of a heterogeneous procedure in order to separate the bound from the unbound portion of the labeled component. In this type of procedure the label can be easily determined. A disadvantage is, however, the long duration of the heterogeneous reaction and the washing step(s) required.

In the homogeneous procedure the bound label is not separated from the unbound label so that differentiation of bound and unbound label must be carried out by other methods. Many different ways are known to set up such assays, amongst which competitive homogenous immunoassays are widely used.

A disadvantage of many competitive homogeneous agglutination immunoassays is that a very time-consuming, parameter-specific optimization of the raw materials is necessary. In all these tests there are mutually opposing requirements for optimal differentiation and optimal sensitivity, since on the one hand the concentration of reagent containing the agglutinating particles should be limited in order to facilitate the competitive reaction with the sample and on the other hand this reagent should be used in a high concentration and highly labeled in order to achieve a sufficient change in agglutination and signal per unit time. Matching these requirements lead to limited sensitivity and to susceptibility to interferences which can often only be eliminated by specific sample pretreatment.

The problem of optimizing all reagents individually for any analyte under investigation has recently been significantly improved by employing assay procedures which are based on the use of an analyte-independent binding pair. This analyte independent binding pair, e.g. the biotin-streptavidin pair is used to mediate the analyte specific reaction to a broadly applicable carrier material, e.g. to latex beads. Details of such methods are especially given in US 5,858,803 and US 5,362,655.

It has been found that in the medium to low concentration range a variety of analytes can be successfully analyzed by homogeneous agglutination assays which are based on the use of polymeric streptavidin, especially of streptavidin coated latex beads. These multivalent and broadly applicable (multipurpose) carrier reagents have big advantages, e.g. they can be produced in big amounts and test development based on them is in general more easy and cheaper, since at least with respect to the carrier, similar problems and solutions apply for these testsystems despite the use of different analyte-specific reagents for detection of different analytes.

Test-systems for different analytes based on multivalent, and especially multipurpose carriers, however, also have one major disadvantage. The ease of use and their broad applicability for different analytes often goes to the expense of a reduced range of analyte concentrations which can be covered by test systems based on such multivalent carriers. In other words, it has proven difficult to use the same multivalent carrier both for the detection of analytes present in low concentrations as well as for detection of analytes present in high concentrations.

This problem is easily explained by the fact that the amount of this multivalent carrier can only be varied to a limited extend, e.g. within the limits given by the hardware of a clinical chemical analyzer. Similar hardware restrictions apply for the volume of sample pipetted. Another problem is the limited availability of immunological reagents like analyte-specific monoclonal antibodies.

Clinically relevant analytes are known to present in samples at quite different concentrations. At the one end of the concentration range analytes, like digoxin, have to be detected, which are present in clinically relevant samples at rather a low concentration and have to be reliably measured down to 1 x 10⁻¹⁰ M. Other analytes, for example phenobarbituric acid or gentamicin are found in a concentration range as high as 3 x 10⁻⁶ M and valproic acid may be as high as 2 x 10⁻⁵ M.

By various steps of assay optimization it has in recent years been possible to demonstrate that analytes present at very low concentrations, e.g. digoxin can be detected by competitive homogeneous immunoassays (Scholer, A., et al., Clin. Chem. 43 (1997) 92-99).

The higher sensitivity at the low end concentration range is only obtained with fully optimized reagents. Especially when multipurpose carriers are used, this has been achieved, however, at the expense of less accurate measurements at the high end concentration range. It therefore has been the task of the present invention to search for and identify a method to improve the measurement of analytes at the high end concentration range of about 10⁻⁶ M using the same multivalent and especially the same multipurpose carrier reagent which successfully can be employed for detection of analytes at very low concentrations, e.g. down to 1 x 10⁻¹⁰ M.

It has been found that this problem can be solved by performing an improved homogeneous assay method for detection of an analyte in a sample based on the analyte-dependent aggregation or agglutination of a multivalent carrier reagent an analyte-derivative bound or capable of binding to said carrier and an analyte-specific binding partner (R1), characterized in that said binding partner (R1) is oligomerized. This way it is possible to adjust the calibration curve and the corresponding measuring range for higher concentrated analytes, allowing for their precise and reliable measurement using the same multivalent or multipurpose carrier as employed for the sensitive detection of an analyte present in low concentration.

Summarizing, the present invention relates to an improved, homogeneous assay method for detection of an analyte in a sample based on the analyte-dependent aggregation or agglutination of a multivalent carrier reagent, an analyte-derivative bound or capable of binding to said carrier and an analyte-specific binding partner (R1), characterized in that said binding partner (R1) is oligomeric. The oligomeric form of (R1) is either obtained by cross-linking (R1) monomers chemically, or (R1) is oligomerized by a reagent (R2) having at least two binding sides for (R1).

Analytes known to be present at rather a high concentration in certain clinically relevant samples can now be measured more accurately, and such measurement requires smaller amounts of costly immunological reagents.

The present invention also comprises the use of a multivalent, multipurpose carrier material in homogeneous aggregation or agglutination assays for the reliable measurement of at least two different analytes whose clinically relevant concentration range is at least 100-fold apart, based on the analyte-dependent aggregation or agglutination of said carrier reagent, an analyte-derivative capable of binding to said carrier and an analyte-specific binding partner (R1), characterized in that the higher concentrated analyte is measured in the presence of a binding partner (R1) which is oligomeric or is oligomerized by a reagent (R2) having at least two binding sides for (R1).

### Detailed Description:

The availability of multivalent carrier reagents has greatly facilitated the development of more sensitive assays, especially immunoassays. However, it is a problem to use the same or a similar multivalent carrier reagent both for the measurement of analytes present in rather low concentrations and for the measurement of analytes present in rather high concentrations.

It now has surprisingly been found that this problem can be solved by applying an improved, homogeneous assay method for detection of an analyte in a sample based on the analyte-dependent aggregation or agglutination of a multivalent carrier reagent an analyte-derivative bound or capable of binding to said carrier and an analyte-specific binding partner (R1), characterized in that said binding partner (R1) is oligomeric or is oligomerized by a reagent (R2) having at least two binding sides for (R1).

Many different principles for carrying out homogeneous immunoassays are known in the art (David Wild, The Immunoassay Handbook, 2^{nd} edition (2000) 177 - 194). Broadly used are homogeneous immunoassays which can be evaluated by turbidimetric or nephelometric assessment (Whicher, J. T., et al., Crit Rev Clin Lab Sci (1983) 213-60). Relevant to the present invention are homogeneous immunoassays which make use of a multivalent carrier reagent. Such multivalent carrier reagents are used for reasons like convenience, ease of application, as well as due to the good assay sensitivity which can be achieved employing such reagents (US 5,858,803, US 5,362,655).

A large variety of multivalent carrier reagents has been described in the art. Most relevant are multivalent carrier reagents based on polymeric biomolecules, like proteins, e.g. bovine serum albumin, dextrane and its derivatives, nucleic acids and nucleic acid derivatives or particulate carrier materials, to which one member of a binding pair is coupled or coated.

Preferably, particulate carrier material, especially latex beads with diameters ranging from 50 to 1000 nm are used and the one partner of a binding pair is attached either by chemical coupling or by adsorption.

A special and preferred form of a multivalent carrier is a multipurpose carrier. It is obtained by attachment of one partner of an analyte-independent binding pair to the carrier. This analyte-independent binding pair can be used with great advantage to mediate the binding of an analyte specific binding partner to the carrier. Most preferred the one member of the analyte non-specific or analyte-independent binding pair which is attached to the carrier is capable of binding to a hapten or hapten-like molecule.

Haptens are small molecules which themselves are not immunogenic. However, when conjugated to appropriate carrier molecules, such conjugates may be used to render them immunogenic and to thus to generate an appropriate immune response. Polyclonal as well as monoclonal antibodies against haptens of various kinds are well known in the art. Well known haptens are for example fluoresceine di-nitro-phenyl-phosphate, steroidal hormones, many low molecular weight drugs, digoxin and digoxigenin.

The term hapten-like molecules is used to describe small molecules which are part of a non-immunological binding pair. Well known examples of such binding pairs are amongst others the prosthetic groups of enzymes and the enzyme, substrate derivatives and corresponding enzymes, biotin/avidin, or biotin/streptavidin. Preferably such hapten-like molecules are characterized by a molecular weight of less than 10 kD, more preferred less than 5 kD, more preferred less than 2 kD and most preferred less than 1 kD. Binding pairs comprising a hapten or hapten-like molecule and exhibiting rather high affinity are preferred. Preferably such affinity is at least 10 ⁻⁹ M/l, more preferred 10 ⁻¹⁰ M/l, most preferred 10 ⁻¹¹ M/l or higher.

The "analyte-derivative" according to the present invention is either the analyte itself or an analogue thereof coupled to the second member of the analyte non-specific binding pair. Most preferred the analyte-derivative is a chemical conjugate comprising the analyte or its analogue and a hapten or hapten-like molecule.

"Analyte analogues" comprises amongst others the analyte molecule modified to allow for covalent coupling, as well as more general terms structures which are immunologically equivalent to the analyte. Immunologically equivalent structures are e.g. peptides representing epitopes of a large molecule. Such peptides nowadays are broadly used to substitute for the necessity of providing large and expensive proteins. Also well-known are the so-called mimetic structures, e.g. described in EP 741 869.

The analyte-derivative may be bound to the multivalent carrier before or during the sample is added to the reaction mixture. This multi- or polyvalent carrier is then used to detect the analyte in a sample. The polyvalent carrier is combined with the sample and/or other reagents required to form the reaction mixture at the time the assay is performed. The reagents combined with the sample have been optimized to allow for agglutination or aggregation to an extend which is directly or indirectly correlated to the concentration of analyte present in the sample. Aggregation or agglutination is measured according to standard procedures and devices by performing turbidimetric or nephelometric measurement.

Homogeneous immunoassays for clinical routine applications are designed to require as little handling, e.g. pipetting steps, as possible. This is very convenient to the user and reduces the chances for errors in performing such assays. For the most advanced assays the reaction mixture comprises an analyte-specific binding partner (R1), a multipurpose, multivalent carrier reagent, an analyte derivative which binds to the multipurpose reagent, and the sample to be analyzed.

It now has surprisingly been found that a modulation of the measuring range and a more precise detection of clinically relevant analytes known to be present at higher concentrations in certain relevant samples with the above described homogeneous procedures, is possible by using a binding partner (R1) which is oligomeric or which is oligomerized by a binding partner (R2) having at least two binding sides for (R1).

A preferred embodiment is a homogeneous assay method for detection of an analyte in a sample based on the analyte-dependent aggregation or agglutination of a multivalent carrier reagent, an analyte-derivative bound or capable of binding to said carrier and an analyte-specific binding partner (R1), characterized in that said binding partner (R1) is oligomeric.

The term "oligomeric" is used to illustrate that a binding partner (R1) is chemically cross-linked. Many different procedures known in the art can be applied to chemically cross-link the binding partner (R1). The cross-linked oligomeric molecules are purified by routine molecular chromatography and the appropriate oligomeric fractions are collected and tested in the intended homogeneous assay. The binding partner (R1) is specifically binding to the analyte. Dependent on the type of analyte this binding partner e.g., is a nucleic acid, a ligand to a receptor, a receptor, an antigen, a sugar, a lectin, or an antibody, as well as an equivalent structure thereto or an equivalent fragment thereof.

Preferably, the analyte is of low molecular weight, i.e. smaller than 5000 D. The analyte e.g. may preferably be selected from the list of analytes given in table 1.

In case of analytes with a molecular weight above 5000 D partial structures of such analytes, e.g. peptidic epitopes, may be used to substitute for the necessity of providing an expensive protein. It is preferred that in case of analytes with a molecular weight above 5000 D analogues, e.g. peptides or mimitopes are used which represent the epitopes of interest on such analytes.

In a preferred embodiment the assay is an immunoassay and reagent (R1) is an antibody which is cross-linked chemically or immunologically.

Preferably, antibodies are chemically cross-linked to result in immunoglobulin oligomers of 2 to up to 30 monomeric immunoglobulins. Preferably, the oligomeric immunoglobulin comprises oligomers of 2 to 20 immunoglobulin monomers, more preferred 2 to 10 immunoglobulin monomers and most preferred of 2 to 6 immunoglobulin monomers. Preferably, the immunoglobulin is of the G class of immunoglobulins (IgG).

Chemically cross-linking can be performed by homo- and heterobifunctional reagents according to procedures known in the art, e.g. as described in P. Tijssen, "Laboratory techniques in biochemistry and molecular biology" Chapter 11 (Macromolecule conjugation) (1985) or in more recent editions of this textbook. The skilled artisan will select those reagents which do not effect the antigen binding properties of the antibody polymerized. The cross-linked antibodies are separated according to their size by appropriate chromatographic procedures.

Preferably, the cross-linked (R1) reagent is used in concentration from 0,1 µg/ml to 200 µg/ml, more preferred from 1 µg/ml to 100 µg/ml and most preferred from 2 µg/ml to 50 µg/ml.

A method according to the present invention further characterized by an oligomeric reagent comprising chemically cross-linked immunoglobulin therefore represents another preferred embodiment. Most preferred the cross-linked immunoglobulin is of class G of immunoglobulins (IgG).

A further preferred embodiment is a homogeneous assay method for detection of an analyte in a sample based on the analyte-dependent aggregation or agglutination of a multivalent carrier reagent, an analyte-derivative bound or capable of binding to said carrier and an analyte-specific binding partner (R1), characterized in that said binding partner (R1) is oligomerized by a reagent (R2) having at least two binding sides for (R1).

The term "oligomerized" by a reagent (R2) is used to indicate that monomeric binding partner (R1) is made oligomeric by incubation with (R2). This (R2) reagent has at least two binding sides for (R1). (R2) by binding to (R1) renders the latter oligomeric. By routine experimentation the appropriate concentrations for (R1) and (R2) for the analyte or for the assay under investigation are selected.

Dependent on the analyte to be measured the concentrations of both the (R1) and (R2) reagent may vary from 0,1 µg/ml to 300 µg/ml. In a checker board titration both the optimal concentrations of (R1) and (R2) are selected.

Preferably, the monomeric (R1) reagent which is oligomerized by an at least bivalent (R2) reagent is used in a concentration range between 0,1 µg/ml to 500 µg/ml, also preferred from 1 µg/ml to 200 µg/ml, more preferred from 2 µg/ml to 100 µg/ml and most preferred from 3 µg/ml to 50 µg/ml.

The reagent (R2) is used in relation to (R1). Relative molar concentrations ranging from 10 parts (R1) to 1 part (R2) up to 1 part (R1) to 5 part (R2) are preferred. More preferably the (R2) to (R1)-ratio is in the range of between 5:1 and 1:3, most preferred the (R2) to (R1)-ratio is between 3:1 and 1:2.

Preferably, the reagent (R2) is an immunological reagent comprising a mono- or a polyclonal antibody reactive with (R1). Most preferred (R2) is a polyclonal antibody. Since each antibody has at least two binding sides for its antigen, antibodies to (R1) naturally contain the at least two binding sides required for oligomerization of (R1).

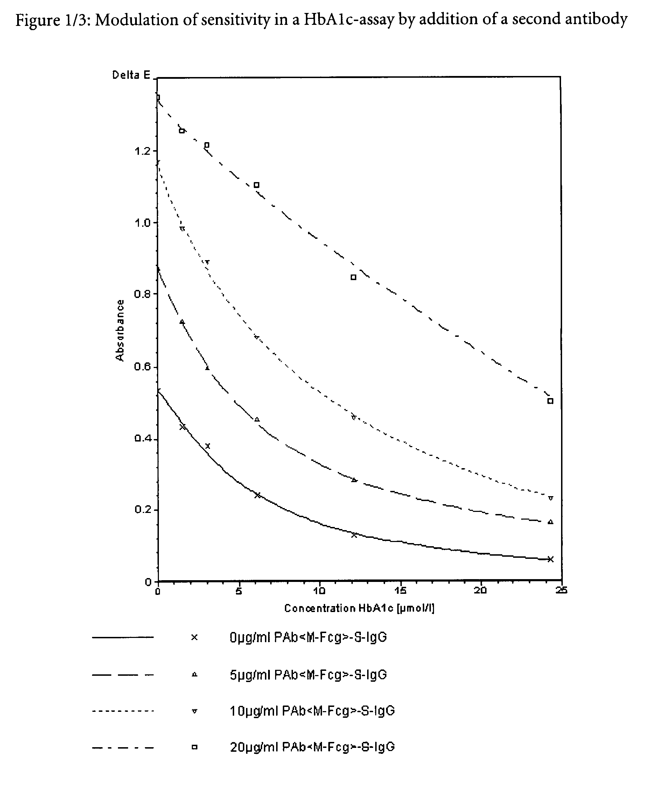

As illustrated in Figures 1 - 3 the use of oligomeric (R1) - independent of whether R1 is oligomerized chemically or by aid of an at least bivalent reagent (R2) - leads to a modulated measurement range which is exemplified by the standard curve shown. A modulated standard curve is less steep in the low concentration range and more steep in the high concentration range.

It is general knowledge in the field that the more steep a standard curve is, the more precise is the measurement in the corresponding concentration range. In the examples given in Figures 1 to 3, the more steep curve in the high concentration range therefore translates into an improved measurement of the more abundant analytes. This is exemplified for two different analytes, HbA1c in Figures 1 and 2 and gentamycin in Figure 3, respectively.

The inventive method is broadly applicable to the measurement of an analyte which is known to occur in samples of interest in concentrations of 10⁻⁷ M or higher.

It is obvious from Table 1 that analytes of diagnostic interest as listed there, are found at quite different concentrations in biological samples. E.g., the requested assay sensitivity (LDL=lower detection limit) ranges from 1,9 x 10⁻¹⁰ M for digoxin to 2.1 x 10⁻⁵ for valproic acid. It has been found that oligomeric (R1) is very useful to measure analytes which require an LDL of 10⁻⁷ or higher. As the skilled artisan appreciates measuring range and LDL of an assay are adjusted to match the concentrations relevant to the clinician.

In a preferred embodiment the present invention is used to assess an analyte which is known to occur in samples of interest in concentrations of 10⁻⁷ M or higher.

It is further preferred to use the method according to the present invention for analytes requiring an LDL in the range of between 2 x 10⁻⁷ and 2 x 10⁻⁵ M.

**Table 1:**

| **LDL required for some clinically relevant analytes** | | |
|---|---|---|
| Analyte | MW | LDL |
| Carbamazepine | 236 | 2.1 x 10⁻⁸ |
| Cyclosporine | 1100 | 1.4 x 10⁻⁸ |
| Digitoxin | 765 | 3.9 x 10⁻⁹ |
| Digoxin | 781 | 1.9 x 10⁻¹⁰ |
| Folat | 441 | 1.4 x 10⁻⁹ |
| Gentamicin | 448 | 5.4 x 10⁻⁷ |
| N-acetylprocaionamide | 277 | 2.2 x 10⁻⁶ |
| Phenobarbital | 231 | 5.2 x 10⁻⁶ |
| Phenytoin | 252 | 2.4 x 10⁻⁶ |
| Procalnamide | 235 | 1.7 x 10⁻⁶ |
| T4 | 777 | 6.4 x 10⁻⁹ |
| Thenophylline | 180 | 4.4 x 10⁻⁶ |
| Tobramycin | 452 | 5.3 x 10⁻⁷ |
| Valproic Acid | 144 | 2.1 x 10⁻⁵ |
| Amphetamine | 135 | 8.1 x 10⁻⁸ |
| Barbiturate | 230 | 8.7 x 10⁻⁹ |
| Benzodiazepine | 284 | 4.6 x 10⁻⁸ |
| Cannabinoide (THC) | 314 | 9.6 x 10⁻⁹ |
| Cocain | 303 | 8.6 x 10⁻⁸ |
| Methadon | 309 | 6.1 x 10⁻⁸ |
| Opiate (Morphin) | 285 | 1.8 x 10⁻⁸ |
| Phencyclidin (PCP) | 243 | 2.5 x 10⁻⁹ |
| MW = molecular weight | | |
| LDL = lower detection limit | | |

In a preferred embodiment the improved homogeneous assay is based on competitive immunoassay procedure. In a competitive type assay the analyte in the sample competes with analyte or analyte analogue bound or capable of binding to the carrier, for a limited number of (R1) binding sites. The more analyte present in the sample, the less (R1) can bind the carrier-bound (or bindable) analyte (or derivative). As a result aggregation or agglutination and analyte-concentration are indirectly correlated.

The inventive method comprising the use of an oligomeric analyte-specific binding partner (R1) is applicable to homogeneous assays based on multivalent carriers, whenever a more accurate measurement in the higher concentration range is required.

In a preferred embodiment the multivalent carrier reagent is a multipurpose reagent coated with a member of an analyte-independent binding pair. Preferably, the inventive procedure is based on a multivalent, multipurpose carrier reagent comprising avidin or streptavidin. Most preferred is streptavidin.

Multivalent, multipurpose carrier material or reagents, e.g. carrier comprising streptavidin, especially latex particles coated with streptavidin are used for a variety of different analytes, with great advantages in assay development and cost reduction. Such multivalent, multipurpose carrier materials based on the present invention can now be used for the reliable detection of different analytes present at quite different concentration in relevant clinical samples. In other terms, these multipurpose carrier materials when used according to the present invention can be used for analytes requiring LDLs at least 100-fold apart. A further preferred embodiment therefore is the use of a multivalent, multipurpose carrier material in a homogeneous aggregation or agglutination assay for the reliable measurement of at least two different analytes whose clinically relevant concentration range is at least 100-fold apart, based on the analyte-dependent aggregation or agglutination of said carrier reagent, an analyte-derivative capable of binding to said carrier and an analyte-specific binding partner (R1), characterized in that the higher concentrated analyte is measured in the presence of a binding partner (R1) which is oligomeric or is oligomerized by a reagent (R2) having at least two binding sides for (R1).

The following examples, references, sequence listing and figures are provided to aid the understanding of the present invention, the true scope of which is set forth in the appended claims. It is understood that modifications can be made in the procedures set forth without departing from the spirit of the invention.

### Description of the Figures

**Figure 1: Modulation of sensitivity in a HbA1c-Assay by addition of a second antibody**
   Various amounts of a polyclonal sheep-anti-mouse-immoglobulin γ chain antibody (=PAb<M-Fcg>S-IgG) have been added into the reaction mixture. A higher calibration curve, e.g. for 20 µg/ml, which is more steep in the higher concentration range, ensures a more precise and more reliable measurement of samples with analyte present at a high concentration, as e.g. compared to measurements read of the steep calibration curve without addition of Pab <MFcg> S-IgG.

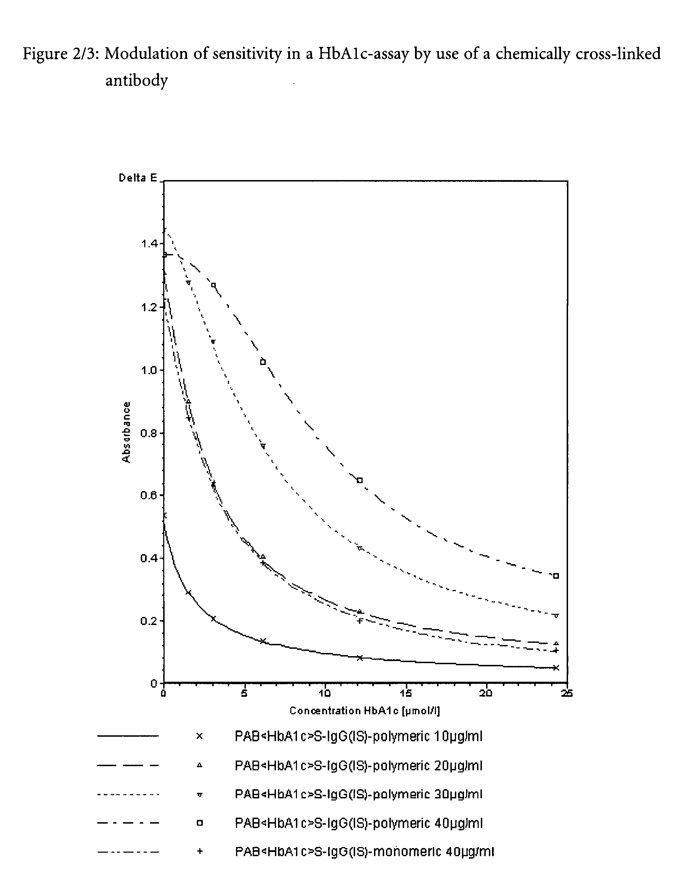

**Figure 2: Modulation of sensitivity in a HbA1c-Assay by use of a chemically cross-linked antibody**
   Polymeric (R1) reagent in this case immunopurified chemically polymerized polyclonal anti-HbA1c antibody from sheep (= PAB <HbA1c> S-IgG(IS)-polymeric) has been added at 10 to 40 µg/ml. It is obvious that in the presence of 30 or 40 µg/ml of polymeric PAB the calibration curve of the assay is significantly different, i.e., modulated as compared to the assay performed with 40 µg/ml of the monomeric (= PAB <HbA1c> S-IgG(IS)-monomeric) R1 antibody.

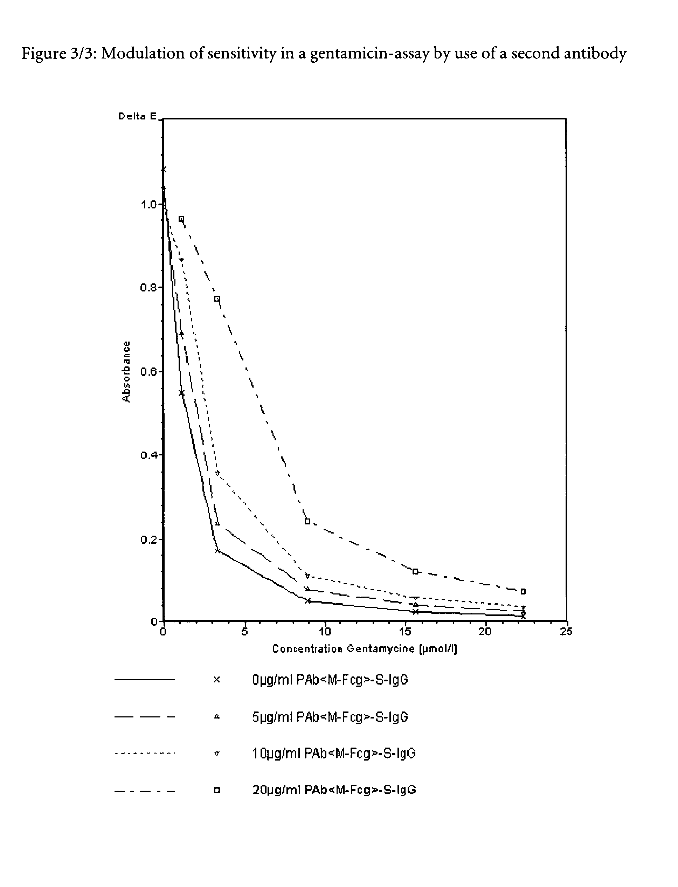

**Figure 3: Modulation of sensitivity in a gentamicin-assay by use of a second antibody**
   Addition of increasing amounts of bivalent (R2) reagent (PAb<M-Fcg>S-IgG) leads to decreased assay sensitivity in the gentamicin as assay shown.

### Example 1: Modulation of sensitivity in a HbA1c-assay by addition of a second antibody

### a) Principle of the SALTINIA technolgoy

Using the SALTINIA technology a HbA1c assay was developed. SALTINIA stands for streptavidin latex turbidimetric inhibition immunoassay. The technology is described in detail in US 5,858,803. In this technology "universal" (i.e. multivalent and multipurpose) SA-coated latex particles with a diameter of about 130nm were used.

The SALTINIA assay is a competitive or inhibition type assay. This means that the analyte in the sample competes with the biotin-analyte conjugate for binding to the bivalent antianalyte antibody. When the biotin-hapten conjugate binds with the antibody, a three dimensional complex is formed. This is because multiple biotin-hapten conjugates are bound through the biotin side to the streptavidin which is coated to the latex particles. The more (competing) analyte is present in the sample the less three dimensional complex or aggregate is formed.

### b) HbA1c-reagents

Antibodies to HbA1c are produced according to procedures as described in US 4,247,533 (polyclonal) or in EP 316 306 and EP 201 187 (monoclonal). A biotinylated glycated HbA1c-peptide (bi-HbA1c), containing the four N-terminal amino acids of the hemoglobin β-chain, a biotinylation at the C-terminus and N-terminally a glycated valine was used. The biotinylated peptide is synthesized according to routine solid phase synthesis procedures, e.g., as described in US 5,804,371 or by Fields and Noble, Int. J. Peptide Res. 35 (1990) 161-214 and thereafter glycated by incubation with a 20-fold molar excess of glucose in 0.2 M sodium phosphate buffer, pH 7,4 for 21 days at room temperature.

### c) Assay procedure

To perform the measurement 2µl of sample or calibrator are pipetted into a cuvette of an Hitachi analyzer and 200µl of a R1-reagent containing the biotinylated HbA1c-conjugate is added and mixed. After 5 minutes 50µl of the second reagent R2 is added, which contains the HbA1c-specific antibody and the SA-coated latex particles. The reaction mixture is stirred immediately and the agglutination reaction is started. After 5 minutes reaction time the turbidity is measured at 546nm wavelength. For calculation of the analyte concentration the difference of the turbidimetric signal at the start of the reaction and the turbidimetric signal after 5 minutes is used.

Following reagents were used:

| R1-reagent: | R2-reagent: |
|---|---|
| 50mM MES buffer, pH 6.6 | 5mM HEPES, pH 7.5 |
| 0.5% detergent | 0,01% preservative |
| 0.1% bovine serum albumin | 40µg/ml MAB-anti-HbA1c |
| 0.01% preservative | 0,25% SA-coated latex particles |
| 0,1µg/ml biotinylated HbA1c-peptide | |
| + polyclonal antibody with anti-mouse-Fcγ-specificity (= PAB<M-Fcγ>S-IgG(IS)) in different concentrations (Fcg in Figure 1 and 3 correspond to Fcγ). | |

By addition of a polyclonal antibody directed against mouse-IgG a multimeric <HbA1c>-antibody aggregate is formed in situ, which is able to enhance the turbidimetric signal of the 0-calibrator and to decrease the slope of the calibration curve (Figure 1).

### Example 2: Cross-linking of IgG from PAb<HbA1c>S-IgG(IS)

Synthetic HbA1c-peptide (cf.: Example 1b) is chemically linked to bovine serum albumin. This hapten-carrier conjugate is used to immunize sheep and the polyclonal sheep anti-HbA1c (= PAb <HbA1c> S-IgG) is immunopurified using the HbA1c-peptide coupled to CNBr-activated sephorose. This polyclonal antibody after immunosorption is termed PAb <HbA1c >S-IgG(IS). 50 mg of PAb < HbA1c >S-IgG(IS) is dissolved in 2 ml 0.05 mol/liter potassium phosphate buffer (pH 7.5) and 0.4 ml disuccinimidyl suberate (manufacturer Pierce) dissolved in dioxan (7.4 mg/ml) is added thereto, with stirring. After incubating for 2 hours at 25°C., the reaction is broken off by the addition of 0.2 ml of 0.1 mole/liter lysine hydrochloride. The reaction batch is diluted with 0.2 ml potassium phosphate buffer (v. supra) and centrifuged. The supernatant is desalinated over an Ultrogel AcA 202 column (LKB, Gräfelfing, Federal Republic of Germany), 11.3 ml being obtained with 45 mg of protein. A part of this preparation is fractionated on a Superose-6-column (Deutsche Pharmacia GmbH) at a flowthrough rate of 0.5 ml/minute in 0.05 mole/liter potassium phosphate buffer (pH 7.0) and the fractions with a molecular weight of about 300,000 to 1,5 million are further used.

### Example 3: Modulation of sensitivity in a HbA1c-assay by use of a chemically crosslinked antibody

The assay conditions are the same as described in Example 1. In this experiment the polymeric anti-HbA1c antibody is not formed by the addition of PAB<M-Fcγ>S-IgG(IS) in the R1-reagent. In this case the monomeric MAB-anti-HbA1c was replaced by a polymeric PAB-anti-HbA1c, produced according to Example 2 in different concentrations. As demonstrated in Figure 2 the slope of the calibration curve has been modulated as required by using this chemically cross-linked polymeric antibody as compared to the same monomeric antibody. E.g., with 30 µg/ml of R1 in the lower concentration range the slope is less steep but still quite appropriate to measure rather low analyte concentrations and in the high analyte concentration range the curve is more steep and thus very appropriate for a precise measurement in that concentration range.

### Example 4: Modulation of sensitivity in a gentamicin assay by use of a second antibody

For this assay also the SALTINIA technology was used. The HbA1c reagents were replaced by gentamicin specific reagents. The assay buffer and assay conditions were slightly changed in comparison to Example 2 and as follows:

| R1-reagent: | R2-reagent: |
|---|---|
| 50mM MES buffer, pH 6.0 | 5mM HEPES, pH 7.5 |
| 150mM NaCl | 0,01% preservative |
| 0.1% detergent | 15µg/ml MAB-anti-gentamicin |
| 0.1% bovine serum albumin | (contained in RDC product 0737844) |
| 0.01% preservative | 0,1% SA-coated latex particles |
| 0,2µg/ml biotinylated gentamicin | |
| + polyclonal antibody with anti-mouse-Fcγ-specificity (PAB<M-Fcγ>S-IgG(IS)) in different concentrations | |

In the gentamicin assay 3µl of sample or calibrator were pippeted into a cuvette of an Hitachi analyzer and 180µl of R1-reagent containing the biotinylated gentamicin was added. After stirring and 5 minutes incubation 80µl of reagent 2 (R2) containing MAB-anti-gentamicin and SA-coated latex particels were added. The reaction mixture is stirred immediately and the agglutination reaction is started. After 5 minutes reaction time the turbidity is measured at 546nm wavelength. For calculation of the analyte concentration the difference of the turbidimetric signal at the start of the reaction and the turbidimetric signal after 5 minutes is used.

In analogy to the HbA1c assay using an oligomeric antibody, in the gentamycin assay a multimeric anti-gentamicin-antibody aggregate is formed *in situ* by addition of the polyclonal antibody directed against mouse-IgG. The *in situ* formed multimeric anti-gentamicin aggregate is able to modulate the slope of the calibration curve as required. This results in a more steep curve for higher concentrations of gentamycin (Figure 3) which translates into a more precise measurement of gentamycin in samples with a high analyte concentration.

### List of References

US 5,362,655
EP 741 869
US 5,858,803
US 5,804,371
EP 201 187
EP 316 306
US 4,247,533
Scholer, A., et al., Clin. Chem. 43 (1997) 92-99
Tijssen, "Laboratory techniques in biochemistry and molecular biology" Chapter 11 (Macromolecule conjugation) (1985)
David Wild, The Immunoassay Handbook, 2^{nd} edition (November 2000) 177 - 194
Whicher, J. T., et al., Crit Rev Clin Lab Sci (1983) 213-60
Fields, G. B., and Noble R. L., Int. J. Peptide Res. 35 (1990) 161-214

## Claims

1. Homogeneous assay method for detection of an analyte in a sample based on the analyte-dependent aggregation or agglutination of a multivalent carrier reagent, an analyte-derivative bound or capable of binding to said carrier and an analyte-specific binding partner (R1), **characterized in that** said binding partner (R1) is oligomeric or is oligomerized by a reagent (R2) having at least two binding sides for (R1).

2. Assay according to claim 1, **characterized in that** said assay is an immunoassay.

3. Assay according to claim 1 or 2, **characterized in that** said oligomeric reagent (R1) comprises chemically cross-linked immunoglobin G (IgG).

4. Assay according to any of claims 1 to 3, **characterized in that** said reagent (R2) is a polyclonal antibody.

5. Assay according to any of claims 1 to 4, **characterized in that** said analyte is known to occur in samples of interest in concentrations of 10⁻⁷ M or higher.

6. Assay according to any of claims 1 to 5, **characterized in that** said homogeneous immunoassay is based on a competitive immunoassay procedure.

7. Assay according to any of claims 1 to 6, **characterized in that** said carrier reagent is a multipurpose reagent.

8. Assay according to any of claims 1 to 7, **characterized in that** said multivalent carrier reagent comprises streptavidin.

9. Use of a multivalent, multipurpose carrier material in a homogeneous aggregation or agglutination assay for the reliable measurement of at least two different analytes whose clinically relevant concentration range is at least 100-fold apart, based on the analyte-dependent aggregation or agglutination of said carrier reagent, an analyte-derivative capable of binding to said carrier and an analyte-specific binding partner (R1), **characterized in that** the higher concentrated analyte is measured in the presence of a binding partner (R1) which is oligomeric or is oligomerized by a reagent (R2) having at least two binding sides for (R1).
